# EUROPEAN PATENT SPECIFICATION

(11) **EP 1 567 103 B1**
(45) Date of publication and mention of the grant of the patent: **13.04.2016**
(21) Application number: 03808335.8
(22) Date of filing: 26.11.2003
(51) Int. Cl.: A61F 13/14, A61F 13/496, A61F 13/15

(54) **PROTECTIVE GARMENT**
SCHUTZKLEIDUNG
ELEMENT DE PROTECTION

(30) Priority: 27.11.2002 US 429647 P
(43) Date of publication of application: 31.08.2005
(73) Proprietor: Tamicare Ltd., Heywood OL10 2TA (GB)
(72) Inventor: GILOH, Tamar, Heywood, OL10 2TA (GB)
(74) Representative: Lane, Cathal Michael
(86) International application number: PCT/IB2003/006472
(87) International publication number: WO 2004/047694

(56) References cited:
- EP-A- 0 737 462
- EP-A- 1 136 050
- WO-A-00/07468
- DE-C- 187 101
- US-A- 2 796 064
- US-A- 4 961 418

## Description

### FIELD OF THE INVENTION

The present invention relates to protective garments generally as well as to methods for manufacture thereof.

### BACKGROUND OF THE INVENTION

Various types of protective undergarments are known in the patent literature. The following patents and patent applications and the references cited therein are believed to represent the state of the art: U.S. Patent 5,098,419; PCT Published Patent Application WO 96/36248 and European Patent 327,823.

WO 00/07468 describes protective undergarments comprising an integrally formed undergarment body formed of a liquid impermeable material. The undergarments described comprise an inner surface and fibres adhered to at least one surface of the undergarment body. A preferred embodiment is described wherein the protective undergarment comprises an absorptive pad.

European Patent No. 1 136 050 describes microporous films comprising flocked fibres, which provide a desirable, breathable material that may be used for example in absorbent articles, wound care products and skin care products. The composite material described may be used in absorbent articles, in particular as a backsheet on sanitary protection articles. It may also be used as a component of a wound care bandage or skin care patch.

### SUMMARY OF THE INVENTION

The present invention seeks to provide a protective garment as set out in the claims, and use of a protective four which is 3D body fitting, body tight, generally lighter and more comfortable than conventional protective garments and which can be manufactured in large quantities at relatively low cost. The protective garment may be worn under other clothing, may be worn in a portion of the body without clothing. As used herein, a "garment" refers to an undergarment or any article to be worn on the body such as a dressing. As used herein, a "dressing" includes, any covering for a sore or wound such as a bandage or any external treatment given through the skin, like pain relievers.

There is thus provided in accordance with one embodiment of the present invention, used of a protective garment as a protective dressing. The protective garment is used in medical applications. The protective garment includes two portions: (1) a garment body (which may be integrally formed and which may be composed of a liquid impermeable material); and (2) an absorptive device or material (such as a pad) associated with the garment body.

Preferably, the garment body may include a first portion which is formed with multiple perforations to permit passage of perspiration therethrough, and a second portion which may be substantially not perforated, so as to prevent passage of body secretions and medical dressings therethrough.

In accordance with one embodiment the absorptive device or material is associated with the garment body at the second portion.

In one embodiment, the second portion extends beyond the absorptive device or material. In an alternate embodiment, the absorptive material or pad may extend beyond the second portion of the garment body.

The protective garment may comprise an integrally formed garment body formed of a liquid impermeable material, the garment body including a first portion which is formed with multiple perforations to permit passage of perspiration therethrough, and a second portion which is substantially not perforated, so as to prevent passage of body secretions and medical dressings therethrough.

Preferably, the liquid impermeable material is a natural elastomer like latex. Alternatively, the liquid impermeable material is artificial elastomer like silicone rubber or polyurethane.

The garment body may have a thickness of less than 300 microns.

The protective garment also comprises an absorbent device or material associated to the garment body.

The protective garment may be a, medical dressing or medical device.

The protective garment has fibers affixed to at least part of one surface of the garment body.

In accordance with one embodiment the absorbent device or material includes a portion extending downward from the crotch along the thigh.

There is additionally provided a method of manufacture of protective garments of the present invention comprising the steps of forming a garment body of a liquid impermeable material, and forming multiple perforations on a first portion of the garment body to permit passage of perspiration therethrough.

Additionally, in accordance with the present invention, the method includes the step of affixing fibers to at least one surface of the undergarment body. For example, cotton fibers may be affixed to at least one surface of the garment body.

The protective garment includes two portions: (1) a garment body and a connector (which may be integrally formed and which may be composed of a liquid impermeable material); and (2) tubing. The garment body may be worn on any portion of the body. One example of the portion of the body may include a portion of the face. Another example of the portion of the body may include a portion of the arm.

The connector may be integral with the garment body. Or, alternatively, the connector may be separate from, but attached to, the garment body. The connector connects the tubing to the protective garment Tubing may be used to absorb or drain liquid from or transmit liquid to the body. The garment body is composed of a liquid impermeable material such as natural and artificial elastomers. Non-limiting examples of synthetic elastomers include polyisobutylene, isobutylene-isoprene copolymer (butyl elastomer), polyvinyl acetate, polyisoprene, polyethylene, vinyl acetate - vinyl laurate copolymers having vinyl laurate contents of about 5% to about 50% by weight of the copolymer, neoprene (polychloroprene), thermoplastic elastomers, nitrile rubber, synthetic latex (which has no proteins), styrenic elastomers, polyurethane, and silicone rubber, and combinations thereof Non-limiting examples of natural elastomers include natural rubber such as smoked or liquid latex and guayule, and combinations thereof. Further, the garment body may include a portion which is formed with multiple perforations to permit passage of perspiration therethrough.

The connector may be composed of a shape-retaining material, such as metal, plastic, a composite (*e.g.* of metal and plastic), or any other suitable material. Non-limiting examples of plastic include polyurethane, polyacrylamide, and polyvinyl chloride (PVC). The connector may have associated with it a material (for example, an absorbent material). The connector may fit with the tubing via a variety of ways, such as by a press fit, by threading on the connector, the tubing or both the connector and the tubing.

A protective garment of the invention can also comprise colors (*e*.*g*. pigments or dyes) and/or fragrances (*e*.*g*. perfumes) in the liquid impermeable material and/or fibers. A color or fragrance can be added, for example, to an elastomeric emulsion before immersing a mold (which will give the protective garment its shape) into the emulsion. Furthermore, a protective garment of the invention can comprise embossed designs or patterns. The designs or patterns can be created by engraving a design in the garment mold. For example, if the protective garment is to be used by children, a teddy bear design can be created in the mold, thereby giving the garment a more attractive appearance to the user. Furthermore, the design can be created with different thicknesses within the same garment (e.g., by further dipping of the desired areas in the liquid latex (either pre- or post-vulcanized) or a synthetic elastomer precursor solution, or by applying coagulants on the mold before dipping, on the desired area to get a thicker result.)

### BRIEF DESCRIPTION OF THE DRAWINGS

The present invention will be understood and appreciated more fully from the following detailed description, taken in conjunction with the drawings in which:
Fig. 1A, 1B, 1C, 1D, 1E, 1F, 1G, 1H and 1I are illustrations of a preferred method for producing protective garments for use in the present invention; and
Fig. 2 is a simplified illustration of protective undergarments produced generally by the method of Figs. 1A - 1F.
Fig. 3A is a simplified illustration of a medical protective garment.
Fig. 3B is a simplified illustration of a medical brassiere (to be used after breast surgery) garments.
Fig. 4 is a simplified illustration of another medical protective garment.
Fig. 5 is a simplified illustration of a medical protective garment with a garment body, a connector and tubing.

### DETAILED DESCRIPTION OF PREFERRED EMBODIMENTS

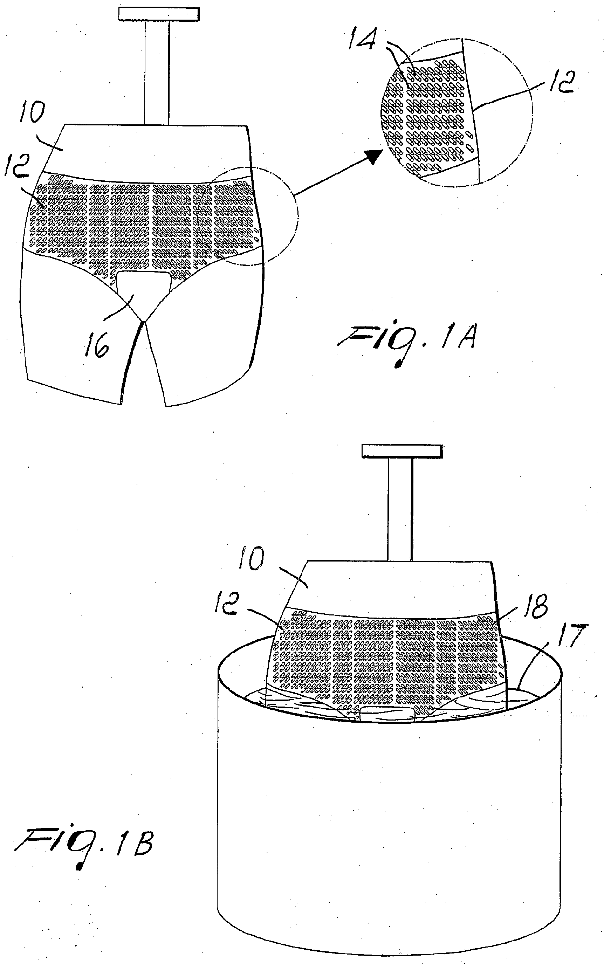

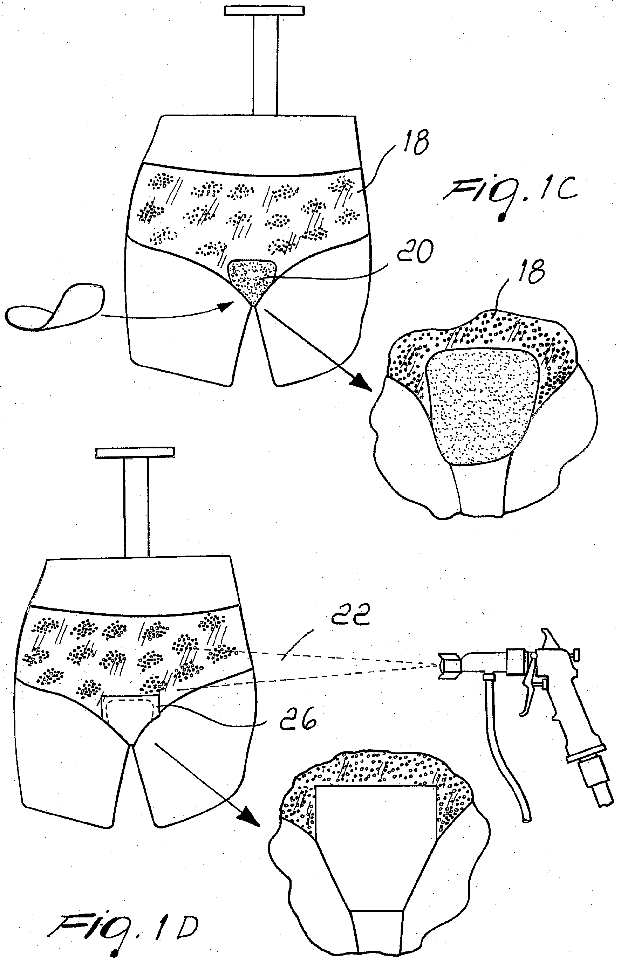

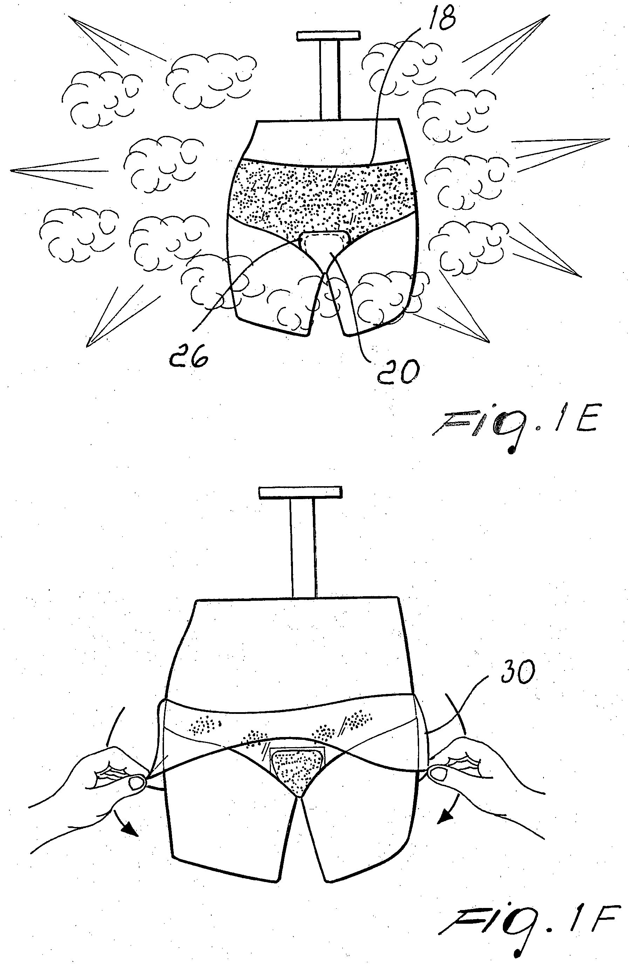

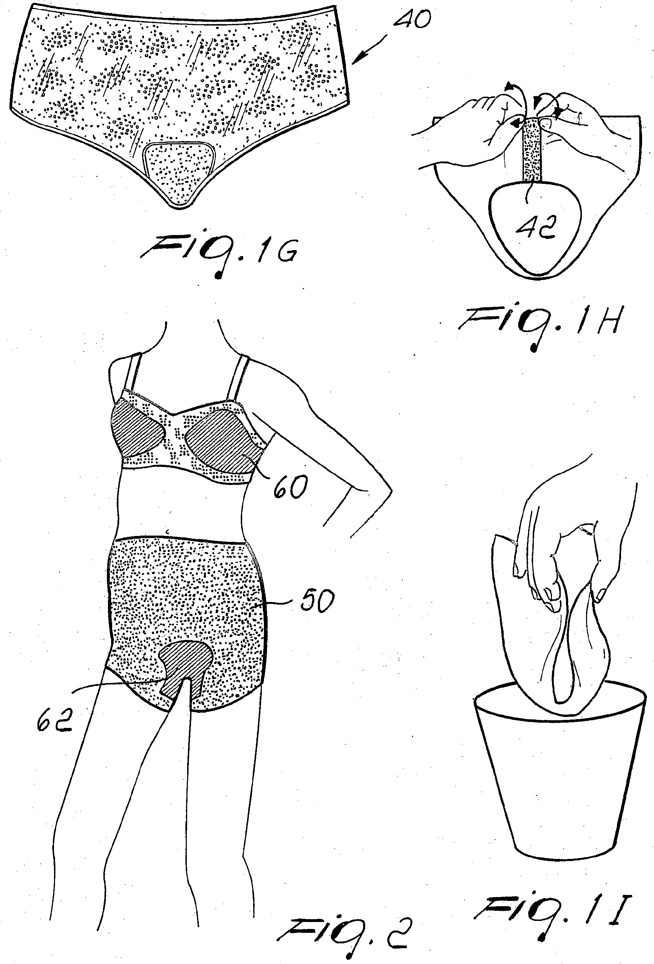

Reference is now made to Fig. 1A, 1H, 1C, 1D, 1E, 1F, 1G, 1H and 1I are simplified illustrations of a method for producing protective garments in accordance with for use in the present invention. The method is described hereinbelow with reference to Figs. 1A- 1I, with specific reference to underpants, it being appreciated that the invention applies equally to any other garment of the present invention (undergarment, or garment worn in conjunction with no other garment).

As seen in Fig. 1A. a three dimensional garment form 10 is provided on which an array 12 of protrusions 14, such as needles, is formed in the general shape of underpants to be formed. The protrusions 14 are preferably covered with TEFLON^{®}. The crotch area 16 of the underpants is preferably not formed with protrusions 14. The remainer of the form 10 is preferably coated with a non-adhesion substance, such as TEFLON^{®}. Otherwise perforations can be made by mechanical means (*e.g.*, roller press).

As shown in Fig. 1B, garment form 10 is preferably dipped in a liquid 17, such as latex or silicone, which when allowed to dry, forms a stretchable, light weight garment 18 over array 12 and crotch area 16. It may be appreciated that the provision of protrusions 14 causes the garment 18 to be perforated except at crotch area 16. Thus the garment 18 is "breathable" except at the crotch area 16, where it is liquid impermeable.

Following dipping of garment form 10 into liquid 17, the garment 18 may be rubbed with a mechanical device (not shown) in order to remove any superfluous elastomer material.

As illustrated in Fig. 1C, an absorptive pad 20 is associated with the light weight garment 18 at the crotch area 16. However, the absorptive pad 20 can be extended beyond the crotch area if desired. It may be associated by the use of an adhesive or attached to the reminder of garment 18 by virtue of the fact that the latex is allowed to dry on the form 10 in engagement with the absorptive pad 20.

Prior to removal of the garment 18 from the form 10, the exterior surface of the garment on the form 10, which will eventually be turned inside-out to form the interior surface, is preferably sprayed with adhesive 22, as shown in Fig. 1D and thereafter with cotton fibers 24 or any other suitable material as indicated in Fig. 1E, thereby to provide a comfortable and non-stick skin engaging surface. Preferably pad 20 is covered during the steps of Figs. 1D and IE with a cover 26, which extends somewhat beyond the pad, so as to prevent liquid migration from the pad to the cotton fibers.

The resulting garment 30 may then be removed from the form 10, as seen in Fig. IF and turned inside out. The outer surface of garment 30 may be coated or otherwise covered with cotton fiber or any other suitable material and finished in an appropriate manner.

The completed garment 40, as seen in Fig. 1G, is preferably extremely light weight, low cost, stretchable, underpants, which is perforated generally except at the specific areas mostly covered with the absorbent material and which is preferably integrally formed with an internal absorptive pad. The garment is preferably formed of an elastic material, such as latex or silicone, and has a thickness of less than 300 microns. As seen in Fig. 1H, a tear region 42 may be incorporated in the garment, such as at a side thereof. This enables ease of removal of the garment, by tearing it at region 42. Once removed the torn, worn garment may be disposed of, as illustrated in Fig. 1I.

The final stage of manufacturing of the garment 18 may include a vulcanization process and edge-cutting of the garment, as known in the art.

As can be seen from Fig. 2, the garment may be underpants 50, a brassiere 60, or any other suitable garment. It is noted that the pad 62 of the underpants 50 has a portion which extend downwardly along the wearer's legs.

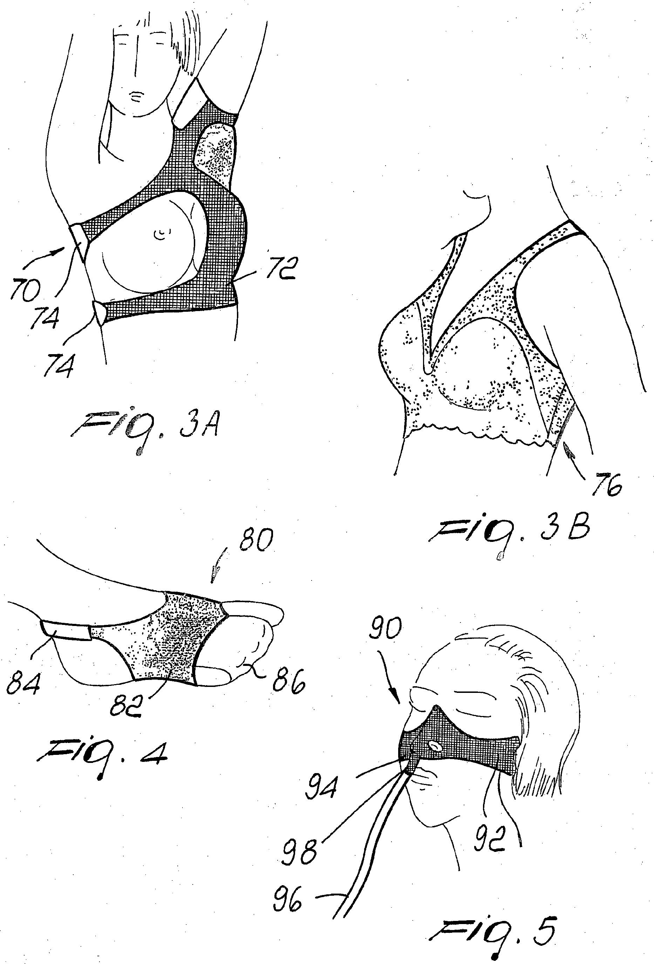

Referring to Fig. 3A, there is shown a medical protection garment 70. The medical protection garment may include a garment body 72. A portion of the garment body (or the entire garment body) may be composed of an elastic material or a liquid impermeable material. Examples of materials may include latex or silicone. The garment body 72 may include straps 74 or other types of adjustment devices (such as snaps, clips, fasteners, or the like) in order to adjust the garment body so that it fits snugly or comfortably with the wearer. Some or all of the garment body may be perforated. A portion of the garment body (not shown in the figure) may be covered with material (such as cotton fibers), thereby to provide a comfortable and non-stick skin engaging surface. The medical protection garment 70 shown in Fig. 3A may be used to protect, bandage and treat a portion of the body, such as single breast, after a medical procedure (such as a breast surgery). The absorptive device or material associated with the garment body may include medical ointments, medicine for treatments or diagnostic materials. Referring to Figure 3B, there is shown an alternate embodiment of the garment 76 showing a simplified illustration of a medical brassiere (to be used after breast surgery) garment.

Referring to Figure 4, there is shown a simplified illustration of another medical protective garment 80. The medical protective garment 80 may include a garment body 82. A portion of the garment body (or the entire garment body) may be composed of an elastic material or a liquid impermeable material. Examples of materials may include latex or silicone. The garment body 82 may include straps 84 or other types of adjustment devices (such as snaps, clips, fasteners, or the like) in order to adjust the garment body so that it fits snugly or comfortably with the wearer. Some or all of the garment body may be perforated. A portion of the garment body (not shown in the figure) may be covered with material such as cotton fibers, thereby to provide a comfortable and non-stick skin engaging surface. As shown in Fig. 4, an absorptive material 86 is attached to garment body 82 while another portion of material 86 may not be attached to garment body 82. The material 86 may be connected at various portions to the garment body. For example, the material 86 may be attached at two of its ends, one to the upper portion of the garment body 82 and the other to the lower portion of the garment body 82. In this manner, a pocket may be formed. The medical protective garment 80 shown in Fig. 4 may be used on a limb, such as a foot, and may help in the treatment of those who suffer from diabetes.

Referring to Fig. 5, there is shown a simplified illustration of a medical protective garment 90 with a garment body 92, a connector 94 and tubing 96. The garment body 92 and the connector 94 may be integrally formed. Alternatively, the garment body 92 and the connector 94 may be composed of separate pieces and may be attached to one another (such as by glue, fasteners or the like). The garment body may be composed of an elastic or liquid impermeable material. Similarly, the connector may be composed of an elastic or liquid impermeable material. The garment body 92 may be worn on any portion of the body. One example of the portion of the body may include on a portion of the face, as shown in Fig. 5. Another example of the portion of the body may include a portion of the arm, such as when a user requires Intravenous liquid.

The garment body 92 may include a portion which is formed with multiple perforations to permit passage of perspiration therethrough. Further, the garment body 92 may include straps (not shown) or other types of adjustment devices (such as snaps, clips, fasteners, or the like) in order to adjust the garment body 92 so that it fits snugly or comfortably with the wearer. In the example shown in Fig. 5, the adjustment device may adjust to fit the head of the user.

The connector 94 connects the tubing 96 to the protective garment 90. Tubing 96 may be used to absorb liquid from or transmit liquid to the body. For example, as shown in Fig. 5, the tubing may be used for drainage from the body. Alternatively, tubing may be used to supply fluid or liquid to the body.

The connector may have associated with it a material (for example, an absorbent material). In this manner, the tubing 96 may be connected to the connector 94 with less discomfort to the user. The connector may fit with the tubing via a variety of ways, such as by threading 98. The threading may be on the tubing 96, on the connector 94 or on both the connector 94 and the tubing 96. Alternatively, the tubing 96 may be fit by a press fitting the tubing 96 with the connector 94.

It will be appreciated by persons skilled in the art of that the present invention is not limited by what has been particularly shown and described hereinabove. Rather the scope of the present invention includes variations and modification of the various features within the scope of the claims.

## Claims

1. A protective garment (40, 50, 60, 70, 76, 80, 90) comprising:
a) an integrally formed garment body of a liquid impermeable material having an inner surface;
b) an absorptive device associated with the integrally formed garment body; and
c) fibers affixed to at least a part of the inner surface of the integrally formed garment body;
**characterised in that** the protective garment is a protective medical dressing in the form of a 3D body fitting, body tight medical protection garment to be worn on the body for medical applications and further comprising a connector (94) associated with the integrally formed garment body; and a tubing (96) connected to the integrally formed garment body by the connector.

2. A protective garment (40, 50, 60, 70, 76, 80, 90) according to claim 1, wherein the connector (94) is associated with an absorptive material.

3. A protective garment (40, 50, 60, 70, 76, 80, 90) according to claim 1 or claim 2, wherein the tubing (96) can absorb or transport fluids away from the body.

4. A protective garment (40, 50, 60, 70, 76, 80, 90) according to any of claims 1 to 3 wherein the tubing (96) can transmit liquid to the body.

5. A protective garment (40, 50, 60, 70, 76, 80, 90) according to any of claims 1 to 4, wherein the connector (94) is integrally formed with the garment body.

6. A protective garment (40, 50, 60, 70, 76, 80, 90) according to any of claims 1 to 5, wherein the connector (94) is separate from, but attached to, the garment body.

7. A protective garment (40, 50, 60, 70, 76, 80, 90) according to any of claims 1 to 6, wherein the connector (94) is composed of a liquid impermeable material, wherein the liquid impermeable material is a natural elastomer or a synthetic elastomer.

8. Use of a protective garment (40, 50, 60, 70, 76, 80, 90) comprising:
a) an integrally formed garment body of a liquid impermeable material having an inner surface;
b) an absorptive device associated with the integrally formed garment body;
c) fibers affixed to at least a part of the inner surface of the integrally formed garment body;
and further comprising a connector (94) associated with the integrally formed garment body; and a tubing (96) connected to the integrally formed garment body by the connector;
as a medical dressing in the form of a 3D body fitting, body tight medical protection garment to be worn on the body for medical applications.

9. Use according to claim 8, wherein the connector (94) is associated with an absorptive material.

10. Use according to claim 8 or claim 9, wherein the tubing (96) can absorb or transport fluids away from the body.

11. Use according to any of claims 8 to 10 wherein the tubing (96) can transmit liquid to the body.

12. Use according to any of claims 8 to 11, wherein the connector (94) is integrally formed with the garment body.

13. Use according to any of claims 8 to 12, wherein the connector (94) is separate from, but attached to, the garment body.

14. Use according to any of claims 8 to 13, wherein the connector (94) is composed of a liquid impermeable material, wherein the liquid impermeable material is a natural elastomer or a synthetic elastomer.

## Patentansprüche

1. Eine Schutzkleidung (40, 50, 60 ,70, 76, 80, 90) die Folgendes aufweist:
a) ein integral geformter Kleidungskörper aus einem flüssigkeitsundurchdringbaren Material mit einer Innenoberfläche;
b) eine absorbierende Vorrichtung assoziiert mit dem integral geformten Kleidungskörper; und
c) an mindestens einem Teil der Innenoberfläche des integral geformten Kleidungskörper befestigte Fasern
**dadurch gekennzeichnet, dass** die Schutzkleidung eine schützende medizinische Bedeckung in der Form einer 3D-körperangepassten körperdichten, medizinischen Schutzkleidung ist, die auf dem Körper für medizinische Anwendungen zu tragen ist, und wobei ferner ein Verbinder (94) vorgesehen ist, der mit dem integral geformten Kleidungskörper assoziiert ist; und
eine Rohrleitung (96) verbunden mit dem integral geformten Kleidungskörper durch den Verbinder.

2. Eine Schutzkleidung (40, 50, 60, 70, 76, 80, 90) gemäß Anspruch 1, wobei der Verbinder (94) mit einem absorbierenden Material assoziiert ist.

3. Eine Schutzkleidung (40, 50, 60 ,70, 76, 80, 90) nach Anspruch 1 oder 2, wobei die Rohrleitung (96) Strömungsmittel vom Körper weg absorbieren oder transportieren kann.

4. Eine Schutzkleidung (40, 50, 60 ,70, 76, 80, 90) nach einem der Ansprüche 1 bis 3, wobei die Rohrleitung (96) Flüssigkeit zum Körper übertragen kann.

5. Eine Schutzkleidung (40, 50, 60 ,70, 76, 80, 90) nach einem der Ansprüche 1 bis 4, wobei der Verbinder (94) integral mit dem Kleidungskörper ausgeformt ist.

6. Eine Schutzkleidung (40, 50, 60 ,70, 76, 80, 90) nach einem der Ansprüche 1 bis 5, wobei der Verbinder (94) vom Kleidungskörper getrennt, aber an diesem angebracht ist.

7. Eine Schutzkleidung (40, 50, 60 ,70, 76, 80, 90) nach einem der Ansprüche 1 bis 6, wobei der Verbinder (94) aus einem flüssigkeitsimpermeabelen Material aufgebaut ist, wobei das flüssigkeitsimpermeabele Material ein natürliches Elastomer oder ein synthetisches Elastomer ist.

8. Verwendung einer Schutzkleidung (40, 50, 60 ,70, 76, 80, 90), die Folgendes aufweist:
a) einen integral geformten Kleidungskörper aus einem flüssigkeitsundurchdringbaren Material mit einer Innenoberfläche;
b) eine absorbierende Vorrichtung assoziiert mit dem integral geformten Kleidungskörper; und
c) Fasern, die befestigt sind an mindestens einem Teil der Innenoberfläche des integral geformten Kleidungskörpers;
und ferner einen Verbinder (94) aufweisend, assoziiert mit dem integral geformten Kleidungskörper und mit einer Rohrleitung (96) verbunden mit dem integral geformten Kleidungsköper durch den Verbinder;
als eine medizinische Bedeckung in der Form einer 3D-körperangepassten körperdichten, medizinischen Schutzkleidung zum Tragen auf dem Körper für medizinische Anwendungen.

9. Verwendung nach Anspruch 8, wobei der Verbinder (94) mit einem absorbierenden Material assoziiert ist.

10. Verwendung nach Anspruch 8 oder 9, wobei die Rohrleitung (96) Fluide vom Körper absorbieren oder weg transportieren kann.

11. Verwendung nach einem der Ansprüche 8 bis 10, wobei die Rohrleitung (96) Flüssigkeit zum Körper übertragen kann.

12. Verwendung nach einem der Ansprüche 1 bis 11, wobei der Verbinder (94) integral mit dem Kleidungskörper geformt ist.

13. Verwendung nach irgendeinem der Ansprüche 1 bis 12, wobei der Verbinder (94) gesondert ist vom, aber befestigt ist am Kleidungskörper.

14. Verwendung nach einem der Ansprüche 8 bis 13, wobei der Verbinder (94) aus einem flüssigkeitsimpermeabelen Material besteht, wobei das flüssigkeitsimpermeabele Material ein natürliches Elastomer oder ein synthetisches Elastomer ist

## Revendications

1. Vêtement de protection (40, 50, 60, 70, 76, 80, 90) comprenant :
a) un corps de vêtement formé d'une seule pièce en un matériau imperméable aux liquides ayant une surface intérieure ;
b) un dispositif absorbant associé au corps de vêtement formé d'une seule pièce ; et
c) des fibres fixées à au moins une partie de la surface intérieure du corps de vêtement formé d'une seule pièce ;
**caractérisé en ce que** le vêtement de protection est un pansement médical de protection ayant la forme d'un vêtement de protection médical serré sur le corps, ajusté au corps en 3D, à porter sur le corps pour des applications médicales et comprenant en outre un connecteur (94) associé au corps de vêtement formé d'une seule pièce ; et un tube (96) connecté au corps de vêtement formé d'une seule pièce par le connecteur.

2. Vêtement de protection (40, 50, 60, 70, 76, 80, 90) selon la revendication 1, dans lequel le connecteur (94) est associé à un matériau absorbant.

3. Vêtement de protection (40, 50, 60, 70, 76, 80, 90) selon la revendication 1 ou la revendication 2, dans lequel le tube (96) peut absorber ou transporter des fluides loin du corps.

4. Vêtement de protection (40, 50, 60, 70, 76, 80, 90) selon l'une quelconque des revendications 1 à 3, dans lequel le tube (96) peut transmettre un liquide au corps.

5. Vêtement de protection (40, 50, 60, 70, 76, 80, 90) selon l'une quelconque des revendications 1 à 4, dans lequel le connecteur (94) est formé d'une seule pièce avec le corps du vêtement.

6. Vêtement de protection (40, 50, 60, 70, 76, 80, 90) selon l'une quelconque des revendications 1 à 5, dans lequel le connecteur (94 est séparé du corps du vêtement, mais attaché à celui-ci.

7. Vêtement de protection (40, 50, 60, 70, 76, 80, 90) selon l'une quelconque des revendications 1 à 6, dans lequel le connecteur (94) est composé d'un matériau imperméable aux liquides, le matériau imperméable aux liquides étant un élastomère naturel ou un élastomère synthétique.

8. Utilisation d'un vêtement de protection (40, 50, 60, 70, 76, 80, 90) comprenant :
a) un corps de vêtement formé d'une seule pièce en un matériau imperméable aux liquides ayant une surface intérieure ;
b) un dispositif absorbant associé au corps de vêtement formé d'une seule pièce ;
c) des fibres fixées à au moins une partie de la surface intérieure du corps de vêtement formé d'une seule pièce ;
et comprenant en outre un connecteur (94) associé au corps de vêtement formé d'une seule pièce ; et un tube (96) connecté au corps de vêtement formé d'une seule pièce par le connecteur ;
en tant que pansement médical ayant la forme d'un vêtement de protection médical serré sur le corps, ajusté au corps en 3D, à porter sur le corps pour des applications médicales.

9. Utilisation selon la revendication 8, dans laquelle le connecteur (94) est associé à un matériau absorbant.

10. Utilisation selon la revendication 8 ou la revendication 9, dans laquelle le tube (96) peut absorber ou transporter des fluides loin du corps.

11. Utilisation selon l'une quelconque des revendications 8 à 10, dans laquelle le tube (96) peut transmettre un liquide au corps.

12. Utilisation selon l'une quelconque des revendications 8 à 11, dans laquelle le connecteur (94) est formé d'une seule pièce avec le corps du vêtement.

13. Utilisation selon l'une quelconque des revendications 8 à 12, dans laquelle le connecteur (94) est séparé du corps du vêtement, mais attaché à celui-ci.

14. Utilisation selon l'une quelconque des revendications 8 à 13, dans laquelle le connecteur (94) est composé d'un matériau imperméable aux liquides, le matériau imperméable aux liquides étant un élastomère naturel ou un élastomère synthétique.
